Europäisches Patentamt

(19) European Patent Office    (11) Publication number: **0 102 422**

Office européen des brevets                    **B1**

⑫                    EUROPEAN  PATENT  SPECIFICATION

(45) Date of publication of patent specification: **29.07.87**    (51) Int. Cl.⁴: **A 61 M 25/00**, B 29 C 47/00

(21) Application number: **82304573.7**

(22) Date of filing: **31.08.82**

(54) **Manufacture of reinforced medico-surgical tubes.**

(43) Date of publication of application:
**14.03.84 Bulletin 84/11**

(45) Publication of the grant of the patent:
**29.07.87 Bulletin 87/31**

(84) Designated Contracting States:
**BE DE FR GB SE**

(56) References cited:
**EP-A-0 036 674
DE-A-2 404 656
DE-A-2 740 063
DE-A-3 006 058
DE-B-2 837 813
FR-A-1 048 288
FR-A-1 363 321
US-A-2 810 424
US-A-4 210 478**

(73) Proprietor: **Sheridan, David S.
Hook Road
Argyle New York 12809 (US)**

(72) Inventor: **Sheridan, David S.
Hook Road
Argyle New York 12809 (US)**

(74) Representative: **Day, Jeremy John et al
REDDIE & GROSE 16 Theobalds Road
London, WC1X 8PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to the manufacture of reinforced catheters, endotracheal tubes, tracheotomy tubes and other medico-surgical tubes and to the resulting products. More particularly, it concerns the production of medico-surgical tubes that have a central body portion containing a helix of wire or other filament that serves to strengthen the tubes to mitigate against kinking of the tubes during use.

Many forms of medico-surgical tubes that comprise a lumen through which fluids may be passed to or from the body of a patient are known. The simplest of these are the catheters (see US—A—3,605,750). More complex medico-surgical tubes include endotracheal tubes (see US—A—3,625,793; 3,725,522 and 3,755,525), post surgical tubes (see US—A—3,589,368), tracheotomy tubes (see US—A—3,975,569), sump drain tubes (see US—A—3,314,430) and oesphageal endoprosthesis tubes. The present invention provides improved methods for the production of any of these and other types of medico-surgical tubes.

It is desirable with most types of medico-surgical tube that they be flexible and have as thin a wall as possible. It has been said by knowledgeable persons that the "the ideal catheter is all lumen and no wall". There is a trade-off, however, in constructing the medico-surgical tube with thin walls and high flexibility, i.e., the thinner and more flexible the tube, the greater the possibility the tube will kink during use. Since kinking can result in complete closure of the lumen with attendant damage or death to the patient, medico-surgical tubes must be structured so as to mitigate kinking.

One way the industry has developed to provide medico-surgical tubes of low kinking potential is to include in them a helix of wire or synthetic filament. Such helix containing tubes are referred to in the trade as "reinforced". In the reinforced tubes, the spacings between coils of the helix are quite small, e.g., 1—2 mm., and are to be contrasted to catheters and like tubes comprising filaments to increase tensile strength, rather than compressive strength (see US—A—2,268,321).

The production of reinforced medico-surgical tubes has been a labor intensive operation.

Not only does the need for much hand labor in the prior known methods of forming reinforced medico-surgical tubes substantially increase the cost of manufacture, but it can lead to nonuniformity in the products. The present invention concerns improved methods for production of reinforced medico-surgical tubes at lower costs and higher degree of uniformity of product than has been possible heretofore.

GB—A—2 043 201 discloses a method for making reinforced medico-surgical tubes in which a continuous inner plastic layer is extruded over a core wire, a wire sheathing is applied on to the inner plastic layer as a continuous structure thereon, the wire sheathing is locally removed at spaced intervals, for example, by a grinding operation thereby providing defined spaced circumferential areas without sheathing and intermediate areas having wire sheathing, an outer plastic layer is applied as a continuous cover over the spaced and intermediate areas to produce a strand structure covered by the outer plastic layer and the strand structure is cut at the region of one axial limitation of each of the spaced areas without wire sheathing to produce strand pieces one end portion of each being without wire sheathing and removing the core wire from the strand pieces.

The method of GB—A—2 043 201 has a number of disadvantages which primarily are derived from the fact that the reinforcement is a continuous wire sheathing which has to be locally removed. Because of this it is essential to operate the method using a core wire to support the tube during the removal step and the core wire subsequently has to be stripped from the final sections of cut tube. It will thus be seen that the method of GB—A—2 043 201 is a labour intensive operation and the apparatus required is therefor complicated by the need to provide rotatable collets and other equipment associated with the removal of the sheathing and means have to be provided for stripping the final tubes from the core wire, unless this is to be done by hand which is, itself undesirable.

A principal object of the present invention is the provision of new methods for the production of reinforced medico-surgical tubes. Further objects include the provision of:

1. Improved reinforced medico-surgical tubes produced by the new methods.

2. Reduction in the cost of manufacture of reinforced medico-surgical tubes.

3. Improvement in uniformity between reinforced medico-surgical tubes of any given type and size.

4. Elimination of handwork in the formation of wire or filament helix as a step in the manufacture of reinforced medico-surgical tubes.

Other objects and further scope of applicability of the present invention will become apparent from the detailed description given hereinafter; it should be understood, however, that the detailed description, while indicating preferred embodiments of the invention, is given by way of illustration only, since various changes and modifications within the scope of the invention as defined by the claims will become apparent to those skilled in the art from this detailed description.

The foregoing objects are accomplished according to the present invention by the production of reinforced medico-surgical tubes by a new method which comprises first extruding a continuous, flexible base tube, cooling the extruded tube, coiling a helix of wire or other filament about the cooled tube, then extruding an outer layer onto the helix and base tube to form a laminated final tube, cutting the final tube into sections of predetermined length and finally forming filament-free ends on the cut sections of final tube.

The filament-free ends may be formed by inserting the end of a cut section of final tube in an injection mold and injection molding the end onto the tube section.

Alternatively, the filament-free end may be created by separately molding or otherwise forming a tube end and welding it onto the end of a cut section of final tube. Advantageously, the welding of the preformed filament-free end is accomplished by induction heating using a ring wafer of tube material containing powered metal.

Another method of forming the filament-free end on the medico-surgical tubes is to provide portions in the final tube that do not contain the filament helix, cutting the final tube into sections through such filament-free portions and shaping such portions into the desired tube end configuration. The filament-free portions in the final tube may be created by either intermittently stopping the coiling of filament helix about the base tube or cutting away portions of filament helix from the base tube before applying the outer layer thereon.

A more complete understanding may be had by reference to the accompanying drawings in which

Fig. 1 is a fragmentary isometric view of an endotracheal tube made in accordance with the invention.

Fig. 2 is an enlarged sectional view taken on the line 2—2 of Fig. 1.

Fig. 3 is an isometric view of one type of a preformed distal end for use in making medico-surgical tubes in accordance with the invention.

Fig. 4 is a lateral view of a catheter made in accordance with the invention.

Fig. 5 is a sectional view taken on the line 5—5 of Fig. 4.

Fig. 6 is a sectional view taken on the line 6—6 of Fig. 4.

Fig. 7 is a lateral view of another embodiment of catheter made in accordance with the invention.

Fig. 8 is a lateral view of yet another embodiment of catheter made in accordance with the invention.

Referring in detail to the drawings, the endotracheal tube 2 comprises a distal end 4 and a proximal end 6 joined together by the central body portion 8. The tube 2 has a major lumen 10 that extends the entire length of the tube and a secondary lumen 12 formed in the wall 14 of tube 2. The secondary lumen 12 opens at its distal end into the balloon cuff 16 and is connected at its proximal end to the inflation tube 18 provided with the pilot balloon 20 and check valve 22.

The central body portion 8 of tube 2 contains a filament helix 24 while the distal end 4 and proximal end 6 are filament-free.

The tube 2 is made of flexible material, e.g., plasticized polyvinyl chloride, polyurethane, silicone rubber or similar material, while the connector element 26 is molded from rigid material, e.g., nylon, acrylic resin, polystyrene or the like. The connector element 26 has a distal end 28 with an O.D. slightly larger than the I.D. of the tube lumen 10 so that the wall 14 of the tube 2 at the proximal end 6 must be slightly expanded to fit over the distal end 28 of element 26. This arrangement holds the element 26 firmly on the tube 2, but requires that the proximal end 6 of tube 2 be free of the filament helix 24 to enable the tube wall 14 to be stretched to fit over the distal end 28 of connector element 26.

Endotracheal tubes such as tube 2 are made in a variety of sizes. Typically such tubes will be about 30 to 40 cm in length with the lumen 10 between about 3 to 10 mm and with an O.D. of about 2 to 3 mm greater than the lumen.

The filament of which the helix 24 is formed is preferably wire having a diameter about 0.1 to 0.5 mm and the spacing between separate coils of wire typically would be about 1 to 3 mm. Various types of wire may be used, but it is advantageous to use tempered wire, e.g., so-called piano wire, especially such wire that is of the stainless steel type. Other types of stiff filaments may be used to form the helix, e.g., nylon monofilaments, polyester filaments and the like.

The production of an endotracheal tube, such as tube 2, according to the invention begins with the extrusion of a continuous tube of flexible material, e.g., plasticized polyvinyl chloride, polyurethane, silicone rubber or the like. This base tube will have a wall thickness of about one-half the thickness desired in the final tube.

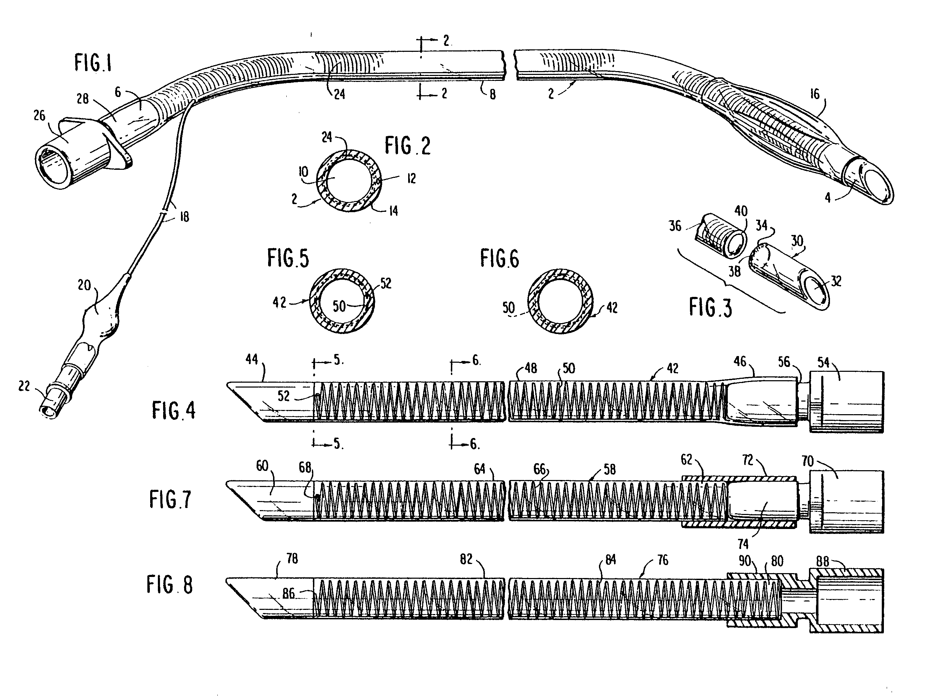

The extruded tube is then cooled by passing it through a cooling bath as it emerges from the extrusion die. Next, a filament is coiled about the cooled tube to form a helix that runs longitudinally along the outer surface of the base tube. A variety of winding machines are available by which such a winding operation may be performed. In one embodiment of the new methods, the helix is continuous along the entire length of the base tube. As such helix wound base tube is formed, it is fed through a second extruder where an outer layer is extruded over the base tube and the helix forming a continuous flexible final tube comprising the helix laminated between the base tube and the outer layer (see Fig. 2). During this second extrusion one or more secondary lumen 12 are formed in the outer layer using a suitable die. The outer layer may be formed oi the same material as the base layer. Alternatively, these may be formed of different materials.

When the resulting final tube is cooled, it is cut into sections of predetermined length after which a filament-free distal end 4 and filament-free proximal end are formed on the tube. This can be accomplished by inserting an end of a cut section of final tube into the cavity of an injection mold and injection molding an end of required shape onto the cut section.

An alternative way of forming filament-free ends on the cut sections of final tube is by preforming a tube end 30 by injection molding, casting or the like. The tube end 30 can have one or more eyes 32 formed therein. Additionally, the end 30 will have in the portion 34 thereof which is to join the cut section 36 of final tube a quantity of powdered metal or equivalent material that will become heated by induction heating. The ring portion 34 may be formed integral with the molded end 30 or it may be a ring wafer separately formed, e.g., by cutting ring sections from an extruded tube made of plastic material containing powdered metal. Thus, when the tube end 38 is positioned to abut the end 40 of final tube section 36 and subjected to a microwave field, the end 30 will be induction welded to the tube section 36. The preformed tube end may be made of the same material as the central body portion 8. Alternatively, they may be made of different material. For example, it may be desirable to have the distal end 4 made of more rigid or more flexible material than the body portion 8. Also, the preformed distal end may contain X-ray opaque material to permit radiology detection of the tube end in the body of a patient.

In another embodiment of the new methods, the cooled base tube is not continuously covered with filament helix prior to applying the outer layer. Thus, the base tube is left free of helix at selected intervals, e.g., helix free portions about 2 to 6 cm in length would be formed between helix covered portions of base tube about 20 to 40 cm in length. These spaced, helix free portions may be created by intermittently stopping the coiling of filament about the base tube. Alternatively, they may be formed by winding a continuous helix onto the base tube and then cutting away portions thereof, for example, by the use of a flying, rotary knife adjusted to cut through the coils of the filament, but not through the base tube.

In this latter embodiment of the invention, the final tube will be cut into sections through the helix free portions and the distal and proximal ends will be formed in the helix free ends of the cut final tube sections in known manner as with unreinforced tubes. Also, the balloon cuffs 16, inflation tube 18 and connector element 26 are applied to the resulting final tube in known manner thereby creating the endotracheal tubes 2.

The distal ends of the medico-surgical tubes of the invention will invariably be free of the filament helix, but this is not true of the proximal ends which in some embodiments may contain the helix. However, the size (length) of the helix free ends may be varied. These variations are illustrated by Figs. 4—8.

The catheter 42 of Fig. 4 comprises a molded, helix-free distal end 44, molded, helix-free proximal end 46 and central body portion 48 containing the helix 50. In order to safeguard against the possibility of the distal end of the helix 50, particularly where this is made of metal wire, penetrating the outer layer of the catheter 42 during its use, a small loop 52 is formed in the distal end of helix 50 prior to forming the molded distal end 44 on the catheter 42.

The rigid molded connector 54 has its distal end 56 encircled by the slightly expanded proximal end 46 of catheter 42.

The catheter 58 of Fig. 7 has a helix free, molded distal end 60, while the proximal end 62 and central body portion 64 contain the helix 66. The distal end 68 of helix 66 is looped in the manner and for the purpose as explained relative to loop 52 in Fig. 4.

The connector 70 is joined to the catheter 58 by means of a segment of flexible, transparent tubing 72 that encircles the proximal end 62 of the catheter 58 and the distal end 74 of connector 70.

The catheter 76 of Fig. 8 comprises a helix free, molded distal end 78, while the proximal end 80 and central body portion 82 both contain helix 84. The distal end 86 of helix 84 is not looped as in the catheter 42 of Fig. 4.

The rigid, molded connector 88 has tubular distal end portion 90 which encircles the proximal end 80 of catheter 76. Cement or adhesive may be applied between parts 80 and 90 to ensure that they do not separate in use.

The new methods disclosed above make it possible to produce reinforced medico-surgical tubes at less cost and with greater uniformity than has been possible heretofore so that they may be treated as one-use, disposable items. The resulting products can be individually packaged and then sterilized, e.g., by exposure to ethylene oxide gas or to cobalt 60 radiation, so that the physician or other user can use the product immediately upon removal of from the sterile package.

**Claims**

1. A method of producing flexible reinforced medico-surgical tubes (2) each having a distal end (4) and a proximal end (6) joined by a central body portion (8) which comprises forming a continuous composite tube comprising an inner flexible base tube, an outer flexible layer laminated to the base tube and a reinforcement (24) between the base tube and the outer layer, cutting said continuous tube into sections of predetermined length, and forming filament-free distal ends (4) upon the cut sections of final tube (2), characterised by the steps of:

extruding an unsupported continuous flexible base tube,

cooling the extruded tube,

coiling a single filament (24) helically about the cooled unsupported continuous tube,

extruding an outer layer of flexible material onto the unsupported base tube and filament helix to form a continuous flexible unsupported final tube (2) comprising said filament helix (24) laminated between said base tube and said outer layer.

2. A method according to claim 1, wherein filament-free proximal end (6) are formed on said cut sections of said final tube (2) in addition to said filament-free distal ends (4).

3. A method according to claim 1 or claim 2, wherein said outer layer comprises a similar material to that of the base tube.

4. A method according to any one of claims 1 to 3, wherein said distal and/or proximal ends (4, 6) are formed by positioning a cut section of final tube (2) as an insert in an injection mold and injection molding said end (4, 6) on an end of said cut section.

5. A method according to any one of claims 1 to 3, wherein said distal and/or proximal ends are formed by providing a preformed filament-free tip (30) and welding said tip (30) to an end of one of said cut sections (36) of final tube (2).

6. A method according to claim 5, wherein said preformed filament-free tip (30) comprises a ring portion (34) at or towards one end containing a quantity of powdered material that will become heated by induction heating and the tip (30) is induction welded onto said final tube (2).

7. A method according to any one of claims 1 to 3, wherein portions of said base tube at spaced intervals remain free of filament helix (24) prior to said extrusion of said outer layer, the cutting to produce said final tubes (2) is performed through said filament-free portions and said filament-free distal ends (4) are formed by shaping of the final tube in the filament-free portion.

8. A method according to claim 7, wherein said portions of said base tube that remain free of filament helix (24) are formed by intermittently stopping the coiling of filament helix (24) about said continuous tube.

9. A method according to claim 7, wherein said portions of said base tube that remain free of filament helix (24) are formed by cutting away portions of filament helix (24) from said base tube prior to extrusion of said outer layer thereon.

10. A method according to any one of claims 1 to 9, wherein the end of the filament helix (24) of a cut section of final tube (2) is bent into a loop (52) before said filament-free distal end (4) is formed and said distal end (4) is formed to envelop said loop (52).

11. A method according to any one of claims 1 to 10, wherein said filament helix (24) is formed from tempered stainless steel wire.

12. A method according to any one of claims 1 to 11, wherein said base tube and said outer layer are formed of plasticised polyvinyl chloride.

**Patentansprüche**

1. Verfahren zum Erzeugen von flexiblen, verstärkten medizinisch chirurgischen Rohren (2), von denen jedes ein distales Ende (4) und ein proximales Ende (6) aufweist, die mit einem zentralen Körperabschnitt (8) verbunden sind, wobei dieser zentrale Körperabschnitt ein kontinuierliches zusammengesetztes Rohr mit einem inneren, flexiblen Basisrohr, einer auf das Basisrohr auflaminierten äußeren flexiblen Schicht und einer Verstärkung (24) zwischen dem Basisrohr und der äußeren Schicht umfaßt, wobei das kontinuierliche Rohr in Abschnitte vorbestimmter Länge geschnitten und nach dem Schneiden fadenfreie distale Enden (4) der geschnittenen Abschnitte des Endrohres (2) gebildet werden, gekennzeichnet durchfolgende Verfahrensschritte:

Extrudieren eines ungestützten, kontinuierlichen, flexiblen Basisrohres,

Kühlen des extrudierten Rohres,

Wickeln eines Einzelfadens (24) schraubenförmig um das gekühlte, ungestützte kontinuierliche Rohr,

Extrudieren eine äußeren Schicht aus flexiblem Material auf das ungestützte Basisrohr und die Fadenwicklung, um ein kontinuierliches, flexibles, ungestütztes Endrohr (2) zu bilden, bei dem die Fadenwicklung (24) zwischen dem Basisrohr und der äußeren Schicht laminiert ist.

2. Verfahren nach Anspruch 1, bei dem die fadenfreien proximalen Enden (6) in Ergänzung zu den fadenfreien distalen Enden (4) an den geschnittenen Abschnitten des Endrohres (2) ausgebildet sind.

3. Verfahren nach Anspruch 1 oder 2, bei dem die äußere Schicht aus einem ähnlichen Material besteht, wie das des Basisrohres.

4. Verfahren nach einem der Ansprüche 1 bis 3, bei dem das distale und/oder proximale Ende (4, 6) dadurch gebildet werden, daß ein geschnittener Abschnitt des Endrohres (2) als Einsatz in eine Spritzgießform positioniert werden und daß dann die Enden (4, 6) an einem Ende des geschnittenen Abschnittes angegossen werden.

5. Verfahren nach einem der Ansprüche 1 bis 3, bei dem das distale und/oder proximale Ende dadurch gebildet wird, daß eine fadenfreie Spitze (30) vorgesehen wird und diese Spitze (30) an ein Ende der geschnittenen Abschnitte (36) des Endrohres (2) geschweißt wird.

6. Verfahren nach Anspruch 5, bei dem die vorgeformte, fadenfreie Spitze (30) an oder in Richtung eines Endes einen Ringabschnitt (34) umfaßt, der eine Menge pulverigen Materials enthält, das durch Induktionsheizen erwärmt wird, und somit die Spitze (30) durch Induktionsschweißen an das Endrohr (2) geschweißt wird.

7. Verfahren nach einem der Ansprüche 1 bis 3, bei dem Bereiche des Basisrohres in Intervallabständen von der Fadenwicklung (24) frei bleiben, bevor das Extrudieren der äußeren Schicht erfolgt, bei dem das Schneiden zum Erzeugen der Endrohre (2) durch die fadenfreien Bereiche vorgenommen wird, und daß die fadenfreien distalen Enden (4) dadurch ausgebildet werden, daß das Endrohr dort die Form eines fadenfreien Abschnittes hat.

8. Verfahren nach Anspruch 7, bei dem die Bereiche des Basisrohres, die von der Fadenwicklung (24) frei bleiben, dadurch gebildet werden, daß intermittierend das Wickeln der Fadenwicklung (24) um das kontinuierliche Rohr angehalten wird.

9. Verfahren nach Anspruch 7, bei dem die Bereiche des Basisrohres, die von der Fadenwicklung (24) frei bleiben, dadurch ausgebildet werden, daß Abschnitte der Fadenwicklung (24) vom Basisrohr weggeschnitten werden, bevor die äußere Schicht darauf extrudiert wird.

10. Verfahren nach einem der Ansprüche 1 bis 9, bei dem das Ende der Fadenwicklung (24) eines geschnittenen Abschnittes des Endrohres (2) in eine Schlaufe (52) geboden ist, bevor das fadenfreie distale Ende (4) ausgebildet wird und bevor das distale Ende (4) gebildet wird, um die Schlaufe (52) zu umgeben.

11. Verfahren nach einem der Ansprüche 1 bis 10, bei dem die Fadenwicklung (24) aus einem Draht gebildet ist, der aus getempertem rostfrien Stahlt besteht.

12. Verfahren nach einem der Ansprüche 1 bis 11, bei dem das Basisrohr und die Außenschicht aus einem plastifizierten Polyvinylchlorid besteht.

## Revendications

1. Procédé de fabrication de tubes médicochirurgicaux renforcés flexibles (2) présentant chacun une extrémité distale (4), ou extrémité avant dans le sens de la pénétration dans le corps, et une extrémité proche (6), ou extrémité arrière considérée dans le même sens, reliées par une partie centrale (8), qui consiste à préparer une tube composite continu comprenant un tube de base intérieur flexible, une couche extérieure flexible stratifiée au tube de base et un renforcement (24) placé entre le tube de base et la couche extérieure, à couper ce tube continu en éléments de longueur prédéterminée et à former des extrémités avant (4) exemptes de filament sur les éléments coupés de tube final (2), caractérisé par les opérations de: extrusion d'un tube de base flexible continu non supporté; refroidissement du tube extrudé; enroulement d'un filament unique (24) hélicoïdalement autour du tube continu non supporté refroidi; extrusion d'une couche extérieure de matière flexible sur le tube de base non supporté et l'hélice de filament pour constituer un tube final non supporté flexible continu (2) comprenant ladite hélice de filament (24) emprisonnée entre ledit tube de base et ladite couche extérieure.

2. Procédé suivant la revendication 1, dans lequel des extrémités arrière (6) exemptes de filament sont formées sur lesdits éléments coupés de tube final (2) en plus des dites extrémités avant (4) exemptes de filament.

3. Procédé suivant la revendication 1 ou la revendication 2, dans lequel ladite couche extérieure est en matière semblable à celle du tube de base.

4. Procédé suivant l'une quelconque des revendications 1 à 3, dans lequel lesdites extrémités avant et/ou arrière (4, 6) sont formées par positionnement d'un élément coupé de tube final (2) comme un insert dans un moule d'injection, et moulage par injection de ladite extrémité (4, 6) sur une extrémité dudit élément coupé.

5. Procédé suivant l'une quelconque des revendications 1 à 3, dans lequel lesdites extrémités avant et/ou arrière sont formées par préparation d'un embout préformé (30) exempt de filament et soudage de cet embout (30) à une extrémité de l'un desdits éléments coupés (36) de tube final (2).

6. Procédé suivant la revendication 5, dans lequel ledit embout préformé (30) exempt de filament comprend une partie annulaire (34) placée à une extrémité ou vers une extrémité et contenant une quantité de matière en poudre qui s'échauffe par chauffage par induction, et l'embout (30) est soudé par induction sur ledit tube final (2).

7. Procédé suivant l'une quelconque des revendications 1 à 3, dans lequel des parties du tube de base à intervalles espacés restent exemptes d'hélice de filament (24) avant ladite extrusion de ladite couche extérieure, la coupe pour obtenir les tubes finals (2) est effectuée à travers lesdites parties exemptes de filament, et les dites extrémités avant exemptes de filament sont obtenues par formage du tube final dans la partie exempte de filament.

8. Procédé suivant la revendication 7, dans lequel lesdites parties du tube de base qui restent exemptes d'hélice de filament (24) sont formées par arrêt intermittent de l'enroulement de l'hélice de filament (24) autour dudit tube continu.

9. Procédé suivant la revendication 7, dans lequel lesdites parties du tube de base qui restent exemptes d'hélice de filament (24) sont formées par coupe et enlèvement de parties d'hélice de filament (24) du tube de base, préalablement à l'extrusion de ladite couche extérieure sur celui-ci.

10. Procédé suivant l'une quelconque des revendications 1 à 9, dans lequel l'extrémité de l'hélice de filament (24) d'un élément coupé de tube final (2) est pliée en une boucle (52) avant de former ladite extrémité avant (4) exempte de filament, et cette extrémité avant (4) est formée de manière à envelopper ladite boucle (52).

11. Procédé suivant l'une quelconque des revendications 1 à 10, dans lequel ladite l'hélice de filament (24) est en fil d'acier inoxydable trempé.

12. Procédé suivant l'une quelconque des revendications 1 à 11, dans lequel ledit tube de base et ladite couche extérieure sont en chlorure de polyvinyle plastifié.

0 102 422

FIG. 1

FIG. 2

FIG. 3

FIG. 4

FIG. 5

FIG. 6

FIG. 7

FIG. 8